(19) Europäisches Patentamt
European Patent Office
Office européen des brevets

(11) Veröffentlichungsnummer: **0 124 743**
**B1**

(12) # EUROPÄISCHE PATENTSCHRIFT

(45) Veröffentlichungstag der Patentschrift: **17.11.88**

(51) Int. Cl.⁴: **C 07 D 211/90** // C07C79/46, C07C69/738

(21) Anmeldenummer: **84103305.3**

(22) Anmeldetag: **26.03.84**

(54) **Verfahren zur Herstellung von unsymmetrischen 1,4-Di-hydropyridindicarbonsäureestern.**

(30) Priorität: **05.04.83 DE 3312283**

(43) Veröffentlichungstag der Anmeldung: **14.11.84 Patentblatt 84/46**

(45) Bekanntmachung des Hinweises auf die Patenterteilung: **17.11.88 Patentblatt 88/46**

(84) Benannte Vertragsstaaten: **CH DE FR GB IT LI SE**

(56) Entgegenhaltungen:
**DE - A - 2 210 672**
**DE - A - 2 335 466**
**DE - A - 2 549 568**
**DE - A - 2 841 667**

**ORGANIC REACTIONS, Band 15, 1967, Seiten 204, 206, 265, 271, 487, John Wiley & Sons; G. JONES "The Knoevenagel condensation"**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Teller, Werner, Dr., In den Birken 79, D-5600 Wuppertal 1 (DE)**

EP 0 124 743 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft ein chemisch eigenartiges Verfahren zur Herstellung von bekannten unsymmetrischen 1,4-Dihydropyridindicarbonsäureestern.

Mehrere Verfahren zu ihrer Herstellung sind bereits bekannt.

So beschreibt beispielsweise die DE-AS 2 117 573 die einstufige Herstellung von unsymmetrischen Dihydropyridinen durch Umsetzung von Aldehyden mit Ketocarbonsäuren und Enaminocarbonsäureestern. In einem zweistufigen Verfahren werden Nachteile gesehen, da die aus Aldehyden und Ketocarbonsäureestern herstellbaren Yliden-β-Ketocarbonsäureester sehr schwierig und oft nur mit geringen Ausbeuten in reiner Form zu isolieren sind.

Es zeigt sich jedoch, dass die einstufige Herstellung von Dihydropyridinen zu Verunreinigungen führt, die durch Reinigungsverfahren nur schwer zu entfernen sind.

Im Hinblick auf die Verwendung der unsymmetrischen 1,4-Dihydropyridine als Arzneimittel besteht nach wie vor ein Bedürfnis, diese Verbindungen mit einem hohen Reinheitsgrad zur Verfügung zu stellen. So konnten beispielsweise bei der Herstellung von
4-(2′-Nitrophenyl)-2,6-dimethyl-3,5-dicarbethoxy-1,4-dihydropyridin
nach US 3 485 847 sieben Nebenprodukte durch Dünnschichtchromatographie festgestellt werden.

In den älteren deutschen Anmeldungen DE-A-2 841 667, DE-A-2 210 672, DE-A-2 549 568 und DE-A-2 335 466 werden bereits Verfahren zur Herstellung von unsymmetrischen Dihydropyridinestern beschrieben. Auch in Organic Reaction, Vol. 15 (1967) Seiten 204–206, 265, 271 und 478 werden Verfahren zur Herstellung von Dihydropyridinen, die als Knoevenagel-Kondensation bekannt sind, beschrieben. Auch die Verwendung von basischen Katalysatoren ist im Zusammenhang mit dem bekannten COPE-Verfahren vorgeschlagen worden.

Die Erfindung betrifft ein Verfahren zur Herstellung von unsymmetrischen 1,4-Dihydropyridinen der allgemeinen Formel I

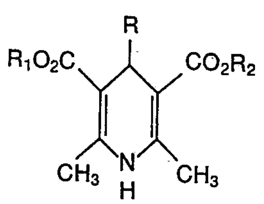

I

in der

R einen Phenylrest darstellt, der gegebenenfalls ein- oder zweifach substituiert ist durch Nitro und/oder Chlor und

$R_1$ einen $C_1$–$C_4$-Alkylrest darstellt, der gegebenenfalls durch eine $C_1$–$C_4$-Alkoxygruppe substituiert ist und

$R_2$ einen $C_1$–$C_{12}$-Alkylrest darstellt, der gegebenenfalls durch eine $C_1$–$C_4$-Alkoxygruppe, eine Trifluormethylgruppe oder den Rest $[C_6H_5CH_2][CH_3]N$ substituiert ist,
wobei die Reste $R_1$ und $R_2$ nicht identisch sind, durch Umsetzung einer Ylidenverbindung der Formeln II oder III

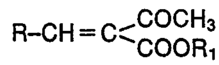

II

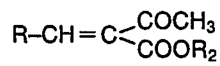

III

mit einer Enaminverbindung der Formeln IV oder V

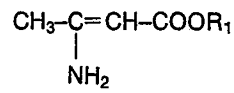

IV

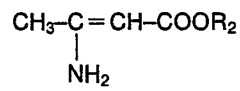

V

dadurch gekennzeichnet, dass man die Ylidenverbindung der Formeln II oder III durch Reaktion eines Ketocarbonsäureesters der Formeln VI oder VII

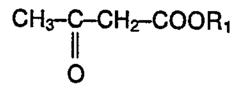

VI

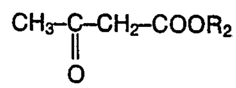

VII

mit einem Aldehyd der Formel RCHO in einem organischen Lösungsmittel (niederer aliphatischer Alkohol) in Gegenwart von katalytischen Mengen an Piperidinacetat bei Temperaturen von −10 °C bis 100 °C herstellt.

Als Lösungsmittel kommen vorzugsweise aliphatische Alkohole wie Methanol, Ethanol und/oder Isopropanol zur Anwendung. Die Umsetzungstemperaturen betragen bevorzugt 20–60 °C.

Der Katalysator wird bevorzugt in Mengen von 0,01 bis 0,7 Mol, besonders bevorzugt von 0,02–0,2 Mol, insbesondere von 0,04–0,2 Mol pro Mol Ketocarbonsäureester zugesetzt.

Pro Mol Ketocarbonsäure der Formel VI können bevorzugt 1 bis 2 Mol, insbesondere 1 Mol Aldehyd eingesetzt werden.

Bevorzugt bedeutet in der Formel I

R einen 2- oder 3-Nitrophenylrest, einen 2- oder 3-Chlorphenylrest oder einen 2,3-Dichlorphenylrest

$R_1$ Methyl, Ethyl, Propyl, Isopropyl, Isobutyl oder einen Propoxyethylrest,

$R_2$ Methyl, Ethyl, Propyl, Isopropyl, Isobutyl, n-Decyl, Methoxyethyl, Propoxyethyl, Trifluormethylmethyl, den Rest $[C_6H_5CH_2][CH_3]N$.

Die Umsetzung der Ylidenverbindung der Formeln II oder III mit der Enaminverbindung der Formeln IV oder V erfolgt bei Temperaturen von −10 bis 130 °C, vorzugsweise von 50 bis 100 °C.

Pro Mol Ylidenverbindung können bevorzugt 1 bis 1,5 Mol, besonders bevorzugt 1 bis 1,3, insbesondere 1 bis 1,2 Mol der Enaminverbindung eingesetzt werden.

Nach einer besonderen Ausführungsform verbleibt die kristalline Ylidenverbindung im Reaktionsgefäss und wird direkt mit der Enaminverbindung umgesetzt.

Es ist als ausgesprochen überraschend zu bezeichnen, dass gemäss der erfindungsgemässen Umsetzung die Yliden-3-ketocarbonsäureester in Gegenwart von den genannten Katalysatoren in grosser Reinheit und exzellenter Ausbeute entstehen und sich sehr gut isolieren lassen.

Weiterhin ist es als überraschend zu bezeichnen, dass auf die beschriebene Art und Weise die 1,4-Dihydropyridinverbindungen in so grosser Reinheit entstehen und isoliert werden können. Sie enthalten – ohne weiteres Reinigungsverfahren – keine Nebenprodukte.

Das erfindungsgemässe Verfahren weist eine Reihe von Vorteilen auf.

So ist die Ausbeute höher als nach den bekannten Verfahren und das isolierte Produkt braucht keinen weiteren Reinigungsschritten unterworfen zu werden.

Die Erfindung sei anhand folgender Beispiele erläutert:

Beispiel 1

a) Ylidencarbonsäureester

Unter Rühren gibt man bei Raumtemperatur zu 325 ml Isopropanol in der folgenden Reihenfolge 80 g (0,5 Mol) Acetessigsäure-2-methoxyethylester, 75,5 g (0,5 Mol) 3-Nitrobenzaldehyd, 1,8 g (0,03 Mol) Eisessig und 2,5 g (0,03 Mol) Piperidin. Man erwärmt auf 40 °C und lässt 30 Minuten bei dieser Temperatur.

Dann kühlt man auf 20 °C ab und rührt 16 Stunden nach. Danach kühlt man auf 0 °C ab und rührt bei dieser Temperatur 1 Stunde nach. Dann wird die überstehende Lösung abgesaugt, das Kristallisat mit 166 ml eiskaltem Isopropanol gewaschen.

Der entstandene 2-(3-Nitrobenzyliden)-acetessigsäure-2-methoxyethylester wird im gleichen Gefäss sofort umgesetzt, wie in b) beschrieben. Isoliert man den 2-(3-Nitrobenzyliden)-acetessigsäure-2-methoxyethylester und trocknet ihn, erhält man 132 g eines hellbraunen Kristallisats (90% der theoret. Ausbeute) vom Schmp. 68–72 °C.

b) Zum nach a) hergestellten, isopropanolfeuchten 2-(3-Nitrobenzyliden)-acetessigsäure-2-methoxyethylester gibt man 270 ml Isopropanol und 64,4 g (0,45 Mol) 3-Aminocrotonsäureisopropylester. Man erhitzt zum Rückfluss (83 °C) und lässt 24 Stunden bei dieser Temperatur.

Nach dem Abkühlen auf 0 °C wird das entstandene Kristallisat isoliert und mit 86 ml Isopropanol gewaschen und trockengesaugt.

Man erhält 173,2 g 4-(3-Nitrophenyl)-2,6-dimethyl-1,4-dihydropyridin-3,5-dicarbonsäure-isopropyl-(2-methoxyethyl)-ester vom Schmelzpunkt 122–127 °C (92% der Theorie).

Die Dünnschichtchromatographie auf Merck-Kieselgelfertigplatten (Laufmittel : Chloroform : Aceton : Petrolether = 3 : 2 : 5) zeigt keine sichtbaren Nebenprodukte.

Beispiel 2

Analog Beispiel 1 erhält man aus dem gemäss Beispiel 1a) hergestellten 3-Nitrobenzylidenacetessigsäureethylester und dem 3-Aminocrotonsäuremethylester nach 10stündigem Erhitzen in ethanolischer Lösung und anschliessendem Stehenlassen über 16 Stunden bei 20 °C den 2,6-Dimethyl-4-(3′-Nitrophenyl)-1,4-dihydropyridine-3,5-dicarbonsäure-3-methylester-5-ethylester der aus Methanol umkristallisiert wird (Fp. 159 °C; Ausbeute 83% der Theorie).

Beispiel 3

a) Ylidencarbonsäureester

Unter Rühren gibt man bei Raumtemperatur zu 325 ml Isopropanol in der folgenden Reihenfolge 58 g (0,5 Mol) Acetessigsäuremethylester, 82,5 g (1,5 Mol) 2,3-Dichlorbenzaldehyd, 1,8 g (0,03 Mol) Eisessig und 2,5 g (0,03 Mol) Piperidin.

Man erwärmt auf 40 °C und lässt 30 Minuten bei dieser Temperatur.

Dann kühlt man auf 20 °C und rührt 16 Stunden nach. Danach kühlt man auf 0 °C ab und rührt bei dieser Temperatur 1 Stunde nach.

Der entstandene 2-(2,3-Dichlorbenzyliden)-acetessigsäuremethylester wird im gleichen Gefäss sofort umgesetzt, wie in b) beschrieben.

b) Zum nach a) hergestellten, 2-(2,3-Dichlorbenzyliden)-acetessigsäuremethylester gibt man 270 ml Isopropanol und 58,9 g (0,45 Mol) 3-Aminocrotonsäureethylester. Man erhitzt zum Rückfluss (83 °C) und lässt 24 Stunden bei dieser Temperatur.

Nach dem Abkühlen auf 0 °C wird das entstandene Kristallisat isoliert und mit 86 ml Isopropanol gewaschen und trockengesaugt.

Man erhält 138 g 4-(2,3-Dichlorphenyl)-2,6-dimethyl-1,4-dihydropyridin-3,5-dicarbonsäuremethyl-ethylester vom Schmelzpunkt 146 °C (80% der Theorie).

**Patentansprüche**

1. Verfahren zur Herstellung von unsymmetrischen 1,4-Dihydropyridinen der Formel I

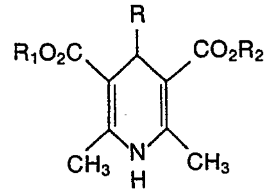

I

in der

R einen Phenylrest darstellt, der gegebenenfalls ein- oder zweifach substituiert ist durch Nitro und/oder Chlor und

$R_1$ einen $C_1$–$C_4$-Alkylrest darstellt, der gegebenenfalls durch eine $C_1$–$C_4$-Alkoxygruppe substituiert ist und

$R_2$ einen $C_1$–$C_{12}$-Alkylrest darstellt, der gegebenenfalls durch eine $C_1$–$C_4$-Alkoxygruppe, eine Trifluormethylgruppe oder den Rest $[C_6H_5CH_2][CH_3]N$ substituiert ist,
wobei die Reste $R_1$ und $R_2$ nicht identisch sind, durch Umsetzung einer Ylidenverbindung der Formeln II oder III

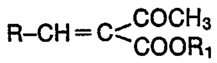 II

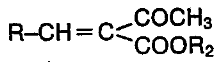 III

mit einer Enaminverbindung der Formeln IV oder V

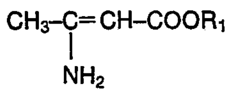 IV

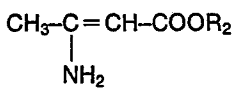 V

dadurch gekennzeichnet, dass man die Ylidenverbindung der Formeln II oder III durch Reaktion eines Ketocarbonsäureesters der Formeln VI oder VII

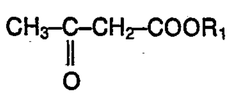 VI

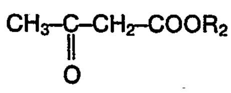 VII

mit einem Aldehyd der Formel RCHO in einem organischen Lösungsmittel (niederer aliphatischer Alkohol) in Gegenwart von katalytischen Mengen an Piperidinacetat bei Temperaturen von −10 °C bis 100 °C herstellt.

2. Verfahren gemäss Anspruch 1, dadurch gekennzeichnet, dass man die Umsetzung zur Herstellung der Ylidenverbindung bei 20–60 °C durchführt.

3. Verfahren gemäss Anspruch 1, dadurch gekennzeichnet, dass man pro Mol des Ketocarbonsäureesters 0,01–0,7 Mol an Piperidinacetat einsetzt.

4. Verfahren gemäss Anspruch 1, dadurch gekennzeichnet, dass man pro Mol Ketocarbonsäureester 1–2 Mol Aldehyd einsetzt.

**Claims**

1. Process for the preparation of unsymmetrical 1,4-dihydropyridines of the formula I

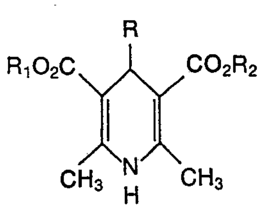 I

in which

R represents a phenyl radical which is optionally mono- or disubstituted by nitro and/or chlorine and

$R_1$ represents a $C_1$–$C_4$-alkyl radical which is optionally substituted by a $C_1$–$C_4$-alkoxy group, and

$R_2$ represents a $C_1$–$C_{12}$-alkyl radical which is optionally substituted by a $C_1$–$C_4$-alkoxy group, a trifluoromethyl group or the radical $[C_6H_5CH_2][CH_3]N$, the radicals $R_1$ and $R_2$ not being identical, by reaction of an ylidene compound of the formulae II or III

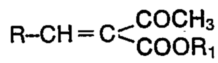 II

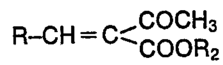 III

with an enamine compound of the formulae IV or V

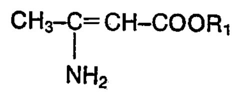 IV

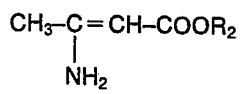 V

characterised in that the ylidene compound of the formulae II or III is prepared by reaction of a ketocarboxylic ester of the formulae VI or VII

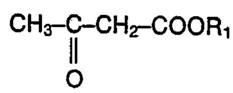 VI

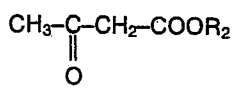 VII

with an aldehyde of the formula RCHO, in an organic solvent (lower aliphatic alcohol), in the presence of catalytic amounts of piperidine acetate, at temperatures from −10 °C up to 100 °C.

2. Process according to Claim 1, characterised in that the reaction for preparing the ylidene compound is carried out at 20–60 °C.

3. Process according to Claim 1, characterised in that 0.01 to 0.7 mole of piperidine acetate are employed per mole of the ketocarboxylic ester.

4. Process according to Claim 1, characterised in that 1 to 2 mole of aldehyde is employed per mole of ketocarboxylic acid.

**Revendications**

1. Procéde de préparation de 1,4-dihydropyridines asymétriques de formule I

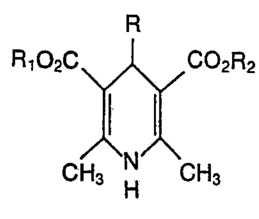 I

dans laquelle

R représente un groupe phényle éventuellement mono- ou di-substitué par des groupes nitro et/ou le chlore, et

$R_1$ représente un groupe alkyle en $C_1$–$C_4$ éventuellement substitué par un groupe alcoxy en $C_1$–$C_4$, et

$R_2$ représente un groupe alkyle en $C_1$–$C_{12}$ éventuellement substitué par un groupe alcoxy en $C_1$–$C_4$, un groupe trifluorométhyle ou le groupe $[C_6H_5CH_2]$ $[CH_3]N$, les groupes $R_1$ et $R_2$ étant différents, par réaction d'un composé en ylidène de formule II ou III

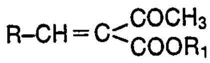
II

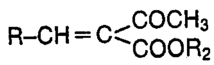
III

avec une énamine de formule IV ou V

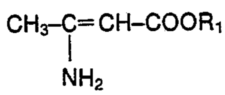
IV

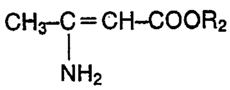
V

caractérisé en ce que l'on prépare le composé en ylidène de formule II ou III par réaction d'un ester d'acide cétocarboxylique de formule VI ou VII

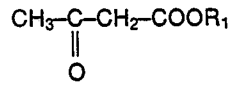
VI

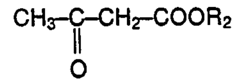
VII

avec un aldéhyde de formule RCHO dans un solvant organique (alcool aliphatique inférieur) en présence de quantités catalytiques d'acétate de pipéridine à des températures de −10 à 100 °C.

2. Procédé selon la revendication 1, caractérisé en ce que la réaction de préparation du composé en ylidène est effectuée à des températures de 20 à 60 °C.

3. Procédé selon la revendication 1, caractérisé en ce que l'on met en œuvre 0,01 à 0,7 mole d'acétate de pipéridine par mole de l'ester d'acide cétocarboxylique.

4. Procédé selon la revendication 1, caractérisé en ce que l'on met en œuvre 1 à 2 moles d'aldéhyde par mole d'ester d'acide cétocarboxylique.